Europäisches Patentamt

(19) European Patent Office

Office européen des brevets

(11) Publication number: **0 142 109 B1**

(12) **EUROPEAN PATENT SPECIFICATION**

(45) Date of publication of patent specification: **17.06.92**

(51) Int. Cl.⁵: **A61K 37/02**, C12P 21/00,
//C12N5/00,(C12P21/00,
C12R1:91)

(21) Application number: **84113229.3**

(22) Date of filing: **05.11.84**

(54) **Macromolecules extracted from cultured mesenchymal cells for the treatment of degenerative diseases.**

(30) Priority: **04.11.83 US 549257**

(43) Date of publication of application:
**22.05.85 Bulletin 85/21**

(45) Publication of the grant of the patent:
**17.06.92 Bulletin 92/25**

(84) Designated Contracting States:
**AT BE CH DE FR GB IT LI NL SE**

(56) References cited:

**NATURE, vol.275, 21 September 1978;
K.M.YAMADA et al., pp.179-184***

**BIOLOGICAL ABSTRACTS, vol.73, no.5, 1982,
Philadelphia, PA (US); A.G.BROWNELL et al.,
p.3140, no.30532***

**BIOLOGICAL ABSTRACTS, vol. 66, no. 11, 01
December 1978, Philadelphia, PA (US);
E.ENGVALL et al., p. 6165, no. 62682***

**CHEMICAL ABSTRACTS, vol. 95, no. 17, 17**

**August 1981, Columbus, OH (US);
W.G.CARTER et al., p. 227, no. 56731t***

(73) Proprietor: **Silverman, Ralph
7701 West Arcadia
Morton Grove Illinois 60053(US)**

(72) Inventor: **Silverman, Ralph
7701 West Arcadia
Morton Grove Illinois 60053(US)**

(74) Representative: **Modiano, Guido et al
MODIANO, JOSIF, PISANTY & STAUB Baader-
strasse 3
W-8000 München 5(DE)**

## Description

It is known from K.M. Yamada et al. Nature, vol. 275, September 21, 1978, pp.179-184, that fibronectin has been found on certain cultured epithelial cells and that fibronectin has a role in cell adhesion.

Furthermore, it is known from Proc. Natl. Acad. Sci. USA, vol. 78, no. 6, 1981, pp.3711-3715 that fibronectin can be obtained from mesenchyme-preconditioned media and that mesenchyme cell-derived fibronectin has possible functions during formation of basal lamina.

This invention relates to a pharmaceutical complex for treating humans and animals with tumors of the epithelial or mesenchymal tissues. The complex comprises a mixture of macromolecules produced from human and animal cultured mesenchymal cells. The mesenchymal cell macromolecular complex consists of fibronectin, procollagen, proteoglycan with or without the macromolecules selected from elastin, laminin and mixtures thereof. Whether or not laminin or elastin are present depends on the particular mesenchymal cell culture used to prepare the complex of my invention.

The invention also relates to a pharmaceutical composition for treating human and animal tumors of the epithelial or mesenchymal tissues, said composition comprising a suitable pharmaceutical carrier and the mesenchymal macromolecular complex of fibronectin, procollagen and proteoglycan, which may further include laminin, elastin or mixtures of laminin and elastin.

The invention also relates to a method of preparing the complex.

For the ease of further describing my invention, I will refer to my composition as PROFIPEL. It is to be understood that this covers those instances where the composition contains all five ingredients - fibronectins, procollagens, proteoglycans, laminins and elastins; four ingredients - fibronectins, procollagens, proteoglycans, and either elastins or laminins; or just three ingredients - fibronectins, procollagens and proteoglycans.

The macromolecules extracted from cultured mesenchymal cells, the PROFIPEL, function with each other in consort and not individually. Therefore, they must not be considered as individual ingredients but must be considered as a complex of mesenchymal macromolecules.

Preferably, the macromolecules in the complex have the same quantitative relationship as those in the mesenchymal cells from which they were extracted.

Preferably, the complex is obtained from healthy mesenchymal cells which underlie healthy epithelial tissue of the counterpart that is diseased.

The PROFIPEL is placed in a therapeutically useful form for administration to the ill animal or human and is particularly useful in treating tumors which affect the epithelial tissue and induces a differentiated state of the cells thereof.

These tumors are mainly treated with drugs and radiation treatment. Such treatment is foreign to the physiological system. The exact manner in which many of these treatments work upon the diseased state is unknown, and their use is based upon trial-and-error observations and studies on humans and animals.

The problem with drugs and/or radiation therapy is that they usually destroy or injure the healthy cells along with the diseased cells. Further, it is almost impossible to localize the treatment only to the diseased area. Thus, not only are healthy cells in the immediate area affected, but also those outside of the diseased area. By destroying or injuring these healthy cells, these treatments often lead to severe side effects including allergies and immunological breakdown. If the treatment compromises a patient's immune system, other life-threatening diseases such as viral or bacterial pneumonias can occur, and further treatments are undermined by the onset of these complications.

Accordingly, an object of this invention is to avoid the use of foreign agents such as drugs and radiation in the treatment of tumors.

Another object of the invention is to provide a nonantigenic complex of mesenchymal macromolecules which promotes redifferentiation of chronically diseased cells and tissue.

Still another object is to provide a nonantigenic complex which is specific for the particular type of tissue being treated.

Yet another object is to provide a process for extracting and purifying the complex in a form for administration.

My preferred embodiments of this invention will be described by use of macromolecules produced by human mesenchymal tissues underlying epithelial tissues. This, of course, is not intended to limit my invention to pharmaceuticals for humans. The same procedure and compositions are also intended for use on animals. In the case of animals, animal mesenchymal cells are used.

The exact mechanism by which PROFIPEL acts on the diseased tissue is not known. However, it is believed that PROFIPEL tends to redifferentiate the diseased cells and thereby promote the natural generation of healthy cells and prevents further dedifferentiation of healthy cells. The complex of macromolecules are the fibronectins, procollagens, proteoglycans, laminins and elastins referred to as PROFIPEL. These macromolecules act in consort although their exact functions are not completely understood.

In this invention, the therapeutic administration of PROFIPEL is used to promote the redifferentiation of the diseased cells and tissue. The preferred form of the invention provides for the treatment of specific epithelial or mesenchymal tissue by PROFIPEL which has been synthesized from specific mesenchymal tissues. For example, if the diseased tissue is lung tissue, then the PROFIPEL used to treat the disease is preferably collected from cultured lung mesenchymal cells.

By varying the type of mesenchymal cells (i.e. lung, liver, skin, etc.) for a specific diseased epithelium, the components of the PROFIPEL complex will vary. For example, as stated above, laminins and elastins may not be present in certain complexes of PROFIPEL, whereas, fibronectins, procollagens and proteoglycans will always be present. Further, the relative quantitative amounts and molecular weights of the various components of PROFIPEL may vary from site to site.

I obtain my PROFIPEL from the culture medium of various types of healthy normal mesenchymal cells in a similar manner. First, various healthy human mesenchymal tissues, usually specific in situ fibroblasts, are obtained from consenting human donors or from other sources such as The American Type Culture Collection of Rockville, Maryland, which has many types of available normal human fibroblasts frozen in liquid nitrogen.

The normal mesenchymal tissue obtained from human donors, is cut into small pieces and treated with the proteolytic enzyme trypsin. This yields a mixture of single cells or small clumps of cells. The cells are then collected by centrifugation. These cells are inoculated into a culture flask containing a suitable growth medium such as Eagles Minimum Essential Medium (MEM) plus 10% fetal calf serum (FCS) which has been filtered through a 0.22 $\mu$m Millipore filter to eliminate its large molecular weight proteins.

The flask containing the growth medium and the cells is incubated at 37°C in an incubator having a 5% carbon dioxide atmosphere. The flask is incubated until the cell culture reaches confluence, which means that the culture covers the total bottom of the culture flask in a single layer. Usually, this occurs within 3 to 12 days.

The cells are next released from the flask by trypsin and EDTA, which are protein digesting agents. The released cells are then reinoculated into several culture flasks in order to expand the culture, and the above incubation step at 37°C in a 5% carbon dioxide atmosphere is repeated. Preferably, a small portion of the released cells are stored in liquid nitrogen for future culture sources.

If frozen cells from the American Type Culture Collection are utilized, these cells are thawed and the culture is started in the same manner as described above.

The cell lines are further expanded by splitting confluent cultures and transferring the cultures to larger cultural vessels such as roller bottles or cultural appliances such as a Hollow Fiber Prefusion Reactor.

The cultured cells excrete their synthesized PROFIPEL macromolecules into the culture medium. These molecules are excreted into the medium in soluble form. The purpose of expanding the cell lines is to obtain as much culture medium as possible. The culture medium is then processed to isolate the soluble PROFIPEL molecules from the medium.

The medium should be processed and separated from the cell culture in one to four days after confluency is reached. The medium can be processed immediately after collection from the culture, or it can be frozen until processing is performed without any loss of protein activity of the soluble PROFIPEL molecules.

Processing and extraction of the PROFIPEL complex from the medium is accomplished by first centrifuging the medium for one half hour at 15,000 rpm. Centrifugation removes the cell fragments, and the clarified medium is placed onto a G-150 gel chromatography column. The fractions of substrate collected from the void volume of the column will contain the PROFIPEL complex. This void volume of the column can be quantitatively measured by passing blue dextran through the empty column.

The initial protein fractions collected from the void volume are observed on a spectrophotometer at optical density of 280 U.V. The collected fractions are pooled, and these pooled fractions contain the soluble fibronectins, procollagens, proteoglycans, laminins and elastins. Only fractions made from one specific type of mesenchymal tissue are pooled. For example, fractions synthesized from lung mesenchymal tissue are only pooled with other fractions made from cultures of lung cells.

The pooled fractions are then lyophilized (freeze-dried) to a powdered material by drying fractions in a frozen state under high vacuum. The pooled fractions can be placed in the final therapeutic ampules, flasks or other containers for the lyophilization procedure. In this way, the lyophilized powder can be reconstituted without further handling. For example, 10ml of pooled fraction in a container could be lyophilized to approximately 2mg of PROFIPEL. The lyophilized PROFIPEL can later be reconstituted in the same container with the amount of sterile saline needed for the selected dosage.

3

A preferred method of producing the PROFIPEL is to utilize a hollow fiber prefusion reactor which is filled with hollow fiber columns. The preferred column is one made with special plastic fiber columns.

In such preferred method, in which the hollow plastic fiber columns permit the excretion of macromolecules from the cells into a culture medium, the cells are perfused with an appropriate culture medium. The cells are cultured and at an appropriate time removed from the culture medium. The complex is then separated from the culture medium in a conventional manner.

Representative of such method is one wherein the mesenchymal cells are cultured until confluence is reached prior to removal of the culture medium from the reaction vessel.

More particularly, confluent culture of normal human mesenchymal cells are added to a sterilized hollow fiber perfusion reactor. The reactor is kept at approximately 37°C. The culture medium is as described above - MEM, FCS. The medium perfuses the cells. The cells are bathed in the nutrients and when they are confluent the cells give up their PROFIPEL which is excreted into the medium. The medium is removed from the reactor and the PROFIPEL separated from the medium by the above-mentioned gel chromatography method. Fresh medium is added simultaneously. This method permits a constant supply of PROFIPEL without disturbing cultured cells or changing culture vessels.

The following examples illustrate the production of

## EXAMPLE

A culture medium was prepared using one liter of Eagles Minimum Essential Medium (MEM) plus glutamine, plus 10% Fetal Calf Serum (FCS) to which was added 1 ml Penicillin, 0.25 ml Streptomycin, 0.6 ml Genomycin, 0.5 ml Fungizone, 10 ml 10% Sodium Bicarbonate, 10 ml MEM Vitamins and 10 ml non-essential amino acids. To prepare the Fetal Calf Serum (FCS), 100 mls FCS was passed through a 0.22 $\mu$m filter to remove the large molecular weight molecules. After this procedure FCS was added to the MEM.

The above MEM + 10% FCS are the medium used throughout this example.

Frozen human foreskin fibroblasts, obtained from the Department of Microbiology, University of Illinois Medical Center, were thawed in a 16 ounce glass flask to which 20 ml of medium was added. The flask was placed into an incubator at 37°C with a 5% carbon dioxide atmosphere and all subsequent incubations were at the same temperature and atmosphere.

The cells reached confluence in 6 days. The cells were transferred using sterile technique into a 32 ounce glass flask. This was accomplished by discarding the spent medium and adding to the flask 5 mls of 0.4% trypsin in 10% Versene (EDTA) for 45 seconds, which helps detach cells from the flask by digesting the attaching proteins. The trypsin-EDTA mixture was discarded and the small flask was washed repeatedly with 10 ml of medium with an electric pipette in order to release the cells into the 10 ml of medium. When all of the cells were released from the flask, the 10 ml of medium containing the cells were pipetted into a sterile 32 ounce flask containing 30 ml of medium, and returned to the incubator.

The cells reached confluence in 7 days and completely covered the bottom of the flask. The cells were again transferred by the above method and the released cells were divided in half and added to two 32 ounce flasks. The spent medium at this stage was saved and frozen. The culture was eventually expanded to fifty 32 ounce flasks and maintained at that level. Each flask contained 40 ml of medium and at each transfer, the spent medium was saved and stored in the freezer.

The PROFIPEL molecules are excreted into the medium by the fibroblasts and extracted from the stored medium. Two hundred and fifty mls of the frozen spent medium was thawed overnight in a cold room. The medium was centrifuged in a refrigerated centrifuge in six 50 ml centrifuge tubes, at 15,000 rpm. for 30 minutes.

The clarified medium was added to the top of a Sephadex® gel G-150 chromatography column and the filtrate was collected in 20 ml fraction collector test tubes, with approximately 15 mls of fraction per tube. Tubes were monitored on a Beckman Spectrophotometer, OD. 280, for the first appearance of protein. For the column used this occurred at approximately 700 ml. The PROFIPEL molecules, only, are contained in the fractions between 700 and 1000 mls (end of the void volume) . The fractions collected from this 300 ml containing the PROFIPEL were pooled.

The 300 ml of collected soluble PROFIPEL were divided into thirty 20 ml lyophilizing ampules with 10 mls of the solute added to each ampule.

The ampules were quickly frozen by placing them into a dry ice-acetone mixture. Ten ampules of the frozen PROFIPEL were then placed into a 1200 ml lyophilizing flask and immediately put onto a large vacuum lyophilizing apparatus at -55°C. This is repeated for ten more ampules until all ampules of PROFIPEL have been lyophilized. The freeze-drying process takes almost 48 hours. Each 20 ml ampule containing 10 ml of soluble PROFIPEL was freeze-dried to about 1.5 mg of solid PROFIPEL.

The ampules were sealed using sterile glass seal flaming. The ampules were then stored in a freezer until needed for administration.

When administered, ampules are opened and a necessary amount of sterile buffered saline for the selected dose is added to the soluble PROFIPEL. It is now ready for injection.

PROFIPEL can be combined in a gel for topical application, used as an enema, or used in a mist for inhalation therapy.

The compositions of this invention are useful in inhibiting the growth of epithelial tissue diseases caused by dedifferentiating cells. In carrying out my novel control method using the compositions of this invention, PROFIPEL, is dissolved in a suitable pharmaceutical carrier, and the solution is injected into the diseased or dedifferentiating cells. In other applications, the PROFIPEL may be put into a salve for topical application, used as an enema or used in a mist for inhalation therapy. The injections are repeated on a regular basis. This inhibits the growth of dedifferentiated cells in the epithelial tissue and reduces the size of the diseased area by redifferentiating some of the diseased cells.

Experiments designed to show the activity of PROFIPEL in suppressing the growth of diseased cells is demonstrated by the following experiment in which, for illustration purposes only, mice were used as exemplary mammals. The determination was carried out as follows:

S180 Tumors were induced in mice by placing S180 mouse sarcoma tumor cells interperitoneally into twenty-one susceptible mice. The PROFIPEL used to treat the mice in Table I, was prepared according to the example. The mice treated with PROFIPEL (two groups) were injected interperitoneally for six days at dosage levels of 50 mg/kg PROFIPEL, and 10 mg/kg, each in 1 ml saline. The control group was given 1 ml sterile saline i.p. The results are given in Table I. In the table, column 1 gives the route of administration, column 2 gives the dose employed, column 3 shows the number of days the mice were treated with PROFIPEL, column 4 indicates the change in animal weight in grams; column 5 shows the number of days the mice survived; column 6 sets forth the medium survival time; column 7 gives the percentage increase in the life span; and column 8 gives the results as being positive or negative.

### TABLE I

| Route | PROFIPEL Dose MG/KG/DAY | Therapy (DAY) | Day 7 AWC (9) | Survival DAYS | MST | %ILS | Result |
|---|---|---|---|---|---|---|---|
| ip | 0 | 1-6 | +5.3 | 10,10,11, 11,12,12 12,13,14 14,15 | 12 | | |
| ip | 50 | • | 0 | 13,16,18, 19,19 | 18 | 50 | + |
| ip | 10 | • | +0.2 | 8,13,16, 17,17 | 16 | 33 | + |

The tests showed that the PROFIPEL complex increased the life span (ILS) as much as 50% in the treatment of S180 tumors at a 50mg per kilogram level, and as much as 33% at a 10mg per kilogram level.

The advantageS of using the PROFIPEL complex should be apparent. First, since the PROFIPEL is synthesized from human mesenchymal tissue, it is considered a nonantigenic substance to humans. Second, the PROFIPEL will not cause immunological breakdown or damage to healthy human tissue.

**Claims**
**Claims for the following Contracting States : BE, CH, DE, FR, GB, IT, LI, NL, SE**

1. A pharmaceutical complex for treating animal and human tumors of the epithelial or mesenchymal tissues comprising a pharmaceutical complex of macromolecules selected from animal and human

5

mesenchymal cells and wherein the complex of macromolecules comprises the combination of fibronectins, procollagens and proteoglycans and may also contain one or more of laminins and elastins.

2. The complex of claim 1 wherein the macromolecules in the complex have the same quantitative relationship as those in the mesenchymal cells from which they were extracted.

3. The complex of claim 1 or 2 wherein the complex is produced from healthy mesenchymal cells underlying healthy epithelium of the counterpart that is diseased.

4. A process of preparing a pharmaceutical complex of animal or human mesenchymal macromolecules comprising placing mesenchymal cells in a hollow fiber perfusion reactor which contains hollow plastic fiber columns, which will permit the excretion of macromolecules from the cells into a culture medium, perfusing the cells with an appropriate culture medium, which will carry the desired macromolecules, culturing the cells, at an appropriate time removing the culture medium, separating the complex from the culture medium in a conventional manner, said complex comprising a mixture of fibronectin, procollagen and proteoglycans and in some instances further including one or more of elastin and laminin.

5. The process of claim 4 wherein the mesenchymal cells are cultured until confluency is reached and then the culture medium is removed from the reaction vessel.

6. A pharmaceutical composition for treating animal and human tumors of the epithelial or mesenchymal tissues comprising a suitable pharmaceutical carrier and characterized by a mesenchymal cell macromolecular complex wherein the complex contains macromolecules of fibronectin, procollagen and proteoglycan and in some instances further includes one or more of elastin and laminin.

7. The pharmaceutical composition of claim 6 characterized in that the complex is obtained from healthy mesenchymal cells which underlie healthy epithelial tissue of the counterpart that is diseased.

**Claims for the following Contracting State : AT**

1. Use of a pharmaceutical complex for preparing a medicament for treating animal and human tumors of the epithelial or mesenchymal tissues, said complex comprising a pharmaceutical complex of macromolecules selected from animal and human mesenchymal cells wherein the complex of macromolecules comprises the combination of fibronectins, procollagens and proteoglycans and may also contain one or more of laminins and elastins.

2. The use according to claim 1 wherein the macromolecules in the complex have the same quantitative relationship as those in the mesenchymal cells from which they were extracted.

3. The use according to claim 1 or 2 wherein the complex is produced from healthy mesenchymal cells underlying healthy epithelium of the counterpart that is diseased.

4. A process of preparing a pharmaceutical complex of animal or human mesenchymal macromolecules comprising placing mesenchymal cells in a hollow fiber perfusion reactor which contains hollow plastic fiber columns, which will permit the excretion of macromolecules from the cells into a culture medium, perfusing the cells with an appropriate culture medium, which will carry the desired macromolecules, culturing the cells, at an appropriate time removing the culture medium, separating the complex from the culture medium in a conventional manner, said complex comprising a mixture of fibronectin, procollagen and proteoglycans and in some instances further including one or more of elastin and laminin.

5. The process of claim 4 wherein the mesenchymal cells are cultured until confluency is reached and then the culture medium is removed from the reaction vessel.

**Revendications**
**Revendications pour les Etats contractants suivants : BE, CH, DE, FR, GB, IT, LI, NL, SE**

1. Complexe pharmaceutique pour le traitement de tumeurs humaines et animales du tissu épithélial ou mésenchymal, comprenant un complexe pharmaceutique de macromolécules choisies parmi les cellules mésenchymales humaines et animales, et dans lequel le complexe de macromolécules comprend la combinaison de fibropectines, de procollagènes et de protéoglycanes, et peut également contenir une ou plusieurs laminines et élastines.

2. Complexe selon la revendication 1, dans lequel les macromolécules du complexe ont la même relation quantitative que celles présentes dans les cellules mésenchymales dont elles sont extraites.

3. Complexe selon la revendication 1 ou 2, dans lequel le complexe est formé à partir de cellules mésenchymales saines sousjacentes à l'épithélium sain de l'homologue de la partie malade.

4. Procédé pour la préparation d'un complexe pharmaceutique de macromolécules mésenchymales humaines ou animales, consistant à placer les cellules mésenchymales dans un réacteur de perfusion à fibres creuses qui contient des colonnes de fibres creuses en matière plastique, ce qui permet l'excrétion des macromolécules à partir des cellules dans un milieu de culture, à imprégner les cellules avec un milieu de culture approprié qui va véhiculer les macromolécules voulues, à effectuer la culture des cellules, à éliminer le milieu de culture au moment approprié, à séparer le complexe du milieu de culture de manière classique, ledit complexe comprenant un mélange de fibropectines, de procollagènes et de protéoglycanes et dans certains cas, comprenant en outre une ou plusieurs élastines et laminines.

5. Procédé selon la revendication 4, dans lequel les cellules mésenchymales sont cultivées jusqu'à ce que la confluence soit atteinte, et ensuite, le milieu de culture est éliminé du récipient réactionnel.

6. Composition pharmaceutique pour le traitement de tumeurs humaines et animales du tissu épithélial ou mésenchymal, comprenant un véhicule pharmaceutique approprié, et caractérisée par un complexe macromoléculaire de cellules mésenchymales, dans laquelle le complexe contient des macromolécules de fibropectine, de procollagène et de protéoglycane, et dans certains cas, contient en outre une ou plusieurs élastines et laminines.

7. Composition pharmaceutique selon la revendication 6, caractérisée en ce que le complexe est obtenu à partir de cellules mésenchymales saines qui sont sousjacentes au tissu épithélial sain de l'homologue de la partie malade.

**Revendications pour l'Etat contractant suivant: AT**

1. Utilisation d'un complexe pharmaceutique pour la préparation d'un médicament destiné au traitement de tumeurs humaines et animales du tissu épithélial ou mésenchymal, comprenant un complexe pharmaceutique de macromolécules choisies parmi les cellules mésenchymales humaines et animales, dans lequel le complexe de macromolécules comprend la combinaison de fibropectines, de procollagènes et de protéoglycanes, et peut également contenir une ou plusieurs laminines et élastines.

2. Utilisation selon la revendication 1, dans laquelle les macromolécules du complexe ont la même relation quantitative que celles présentent dans les cellules mésenchymales dont elles sont extraites.

3. Utilisation selon la revendication 1 ou 2, dans laquelle le complexe est formé à partir de cellules mésenchymales saines sousjacentes à l'épithélium sain de l'homologue de la partie malade.

4. Procédé pour la préparation d'un complexe pharmaceutique de macromolécules mésenchymales humaines ou animales, consistant à placer les cellules mésenchymales dans un réacteur de perfusion à fibres creuses qui contient des colonnes de fibres creuses en matière plastique, ce qui permet l'excrétion des macromolécules à partir des cellules dans un milieu de culture, à imprégner les cellules avec un milieu de culture approprié qui va véhiculer les macromolécules voulues, à effectuer la culture des cellules, à éliminer le milieu de culture au moment approprié, à séparer le complexe du milieu de culture de manière classique, ledit complexe comprenant un mélange de fibropectine, de procollagène et de protéoglycanes et dans certains cas, comprenant en outre une ou plusieurs élastines et laminines.

EP 0 142 109 B1

**5.** Procédé selon la revendication 4, dans lequel les cellules mésenchymales sont cultivées jusqu'à ce que la confluence soit atteinte, et ensuite, le milieu de culture est éliminé du récipient réactionnel.

**Patentansprüche**

**Patentansprüche für folgende Vertragsstaaten : BE, CH, DE, FR, GB, IT, LI, NL, SE**

**1.** Pharmazeutischer Komplex zur Behandlung von tierischen und menschlichen Tumoren der Epithel- und Mesenchymgewebe, der einen pharmazeutischen Komplex von aus tierischen und menschlichen Mesenchymzellen selektierten Makromolekülen umfaßt, und worin der Komplex von Makromolekülen die Kombination von Fibronektinen, Prokollagenen und Proteoglykanen umfaßt und außerdem eines oder mehrere von Lamininen und Elastinen enthalten kann.

**2.** Komplex nach Anspruch 1, worin die Makromoleküle in dem Komplex die gleiche quantitative Beziehung aufweisen wie jene in den Mesenchymzellen, aus denen sie extrahiert wurden.

**3.** Komplex nach Anspruch 1 oder 2, worin der Komplex von gesunden Mesenchymzellen produziert ist, die dem gesunden Epithel eines Gegenstücks zu dem erkrankten Teil zugrundeliegen.

**4.** Verfahren zur Herstellung eines pharmazeutischen Komplexes von tierischen oder menschlichen mesenchymalen Makromolekülen, bei dem man Mesenchymzellen in einem Hohlfaser-Perfusionsreaktor anordnet, der Kunststoffhohlfasersäulen enthält, die die Exkretion von Makromolekülen aus den Zellen in ein Kulturmedium gestatten, die Zellen mit einem geeigneten Kulturmedium, das als Träger für die gewünschten Makromoleküle dient, perfundiert, die Zellen kultiviert, sowie zu einem geeigneten Zeitpunkt das Kulturmedium entfernt und den Komplex von dem Kulturmedium auf herkömmliche Weise abtrennt, wobei dieser Komplex eine Mischung von Fibronektin, Prokollagen und Proteoglykanen umfaßt und in gewissen Fällen außerdem eines oder mehrere von Elastin und Laminin einschließt.

**5.** Verfahren nach Anspruch 4, worin die Mesenchymzellen kultiviert werden, bis Konfluenz erreicht ist, und daß dann das Kulturmedium aus dem Reaktionsbehälter entfernt wird.

**6.** Pharmazeutische Zusammensetzung zur Behandlung von tierischen und menschlichen Tumoren der Epithel- und Mesenchymgewebe, die einen geeigneten pharmazeutischen Träger umfaßt und die gekennzeichnet ist durch einen Komplex von Mesenchymzellen-Makromolekülen, wobei der Komplex Makromoleküle von Fibronektin, Prokollagen und Proteoglykan enthält und in gewissen Fällen außerdem eines oder mehrere von Elastin und Laminin einschließt.

**7.** Pharmazeutische Zusammensetzung nach Anspruch 6, dadurch gekennzeichnet, daß der Komplex aus gesunden Mesenchymzellen erhalten ist, die dem gesunden Epithelgewebe des Gegenstücks zu dem erkrankten Teil zugrundeliegen.

**Patentansprüche für folgenden Vertragsstaat: AT**

**1.** Verwendung eines pharmazeutischen Komplexes zur Herstellung eines Arzneimittels zur Behandlung von tierischen und menschlichen Tumoren der Epithel- und Mesenchymgewebe, der einen pharmazeutischen Komplex von aus tierischen und menschlichen Mesenchymzellen selektierten Makromolekülen umfaßt, und worin der Komplex von Makromolekülen die Kombination von Fibronektinen, Prokollagenen und Proteoglykanen umfaßt und außerdem eines oder mehrere von Lamininen und Elastinen enthalten kann.

**2.** Verwendung nach Anspruch 1, worin die Makromoleküle in dem Komplex die gleiche quantitative Beziehung aufweisen wie jene in den Mesenchymzellen, aus denen sie extrahiert wurden.

**3.** Verwendung nach Anspruch 1 oder 2, worin der Komplex von gesunden Mesenchymzellen produziert ist, die dem gesunden Epithel eines Gegenstücks zu dem erkrankten Teil zugrundeliegen.

**4.** Verfahren zur Herstellung eines pharmazeutischen Komplexes von tierischen oder menschlichen mesenchymalen Makromolekülen, bei dem man Mesenchymzellen in einem Hohlfaser-Perfusionsreaktor anordnet, der Kunststoffhohlfasersäulen enthält, die die Exkretion von Makromolekülen aus den

8

Zellen in ein Kulturmedium gestatten, die Zellen mit einem geeigneten Kulturmedium, das als Träger für die gewünschten Makromoleküle dient, perfundiert, die Zellen kultiviert, sowie zu einem geeigneten Zeitpunkt das Kulturmedium entfernt und den Komplex von dem Kulturmedium auf herkömmliche Weise abtrennt, wobei dieser Komplex eine Mischung von Fibronektin, Prokollagen und Proteoglykanen umfaßt und in gewissen Fällen außerdem eines oder mehrere von Elastin und Laminin einschließt.

5. Verfahren nach Anspruch 4, worin die Mesenchymzellen kultiviert werden, bis Konfluenz erreicht ist, und daß dann das Kulturmedium aus dem Reaktionsbehälter entfernt wird.